(19) Europäisches Patentamt
European Patent Office
Office européen des brevets

(11) Veröffentlichungsnummer: **0 001 821**
**B1**

# (12) EUROPÄISCHE PATENTSCHRIFT

(45) Veröffentlichungstag der Patentschrift: **29.04.81**

(51) Int. Cl.³: **C 07 C 99/12,** C 07 C 101/00, C 07 C 119/10, C 07 B 19/00

(21) Anmeldenummer: **78101258.8**

(22) Anmeldetag: **28.10.78**

(54) **Verfahren zur Racematspaltung von DL-alpha-Aminocarbonsäuren und dafür verwendete Salze.**

(30) Priorität: **03.11.77 DE 2749203**

(43) Veröffentlichungstag der Anmeldung: **16.05.79 Patentblatt 79/10**

(45) Bekanntmachung des Hinweises auf die Patenterteilung: **29.04.81 Patentblatt 81/17**

(84) Benannte Vertragsstaaten: **BE CH FR GB NL**

(56) Entgegenhaltungen:
**CH-A-593 880**
**DE-A-2 309 180**
**DE-A-2 322 412**
**DE-A-2 400 489**
**US-A-3 458 568**
**US-A-4 005 088**

(73) Patentinhaber: **RIEDEL-DE HAEN AKTIENGESELLSCHAFT, Wunstorfer Strasse 40, D-3016 Seelze 1 (DE)**

(72) Erfinder: **Dannenberg, Wolfgang, Dr., Düendorfer Weg 20, D-3050 Wunstorf (DE)**
Erfinder: **Schmand, Horst, Dr., Riepener Strasse 17, D-3052 Bad Nenndorf (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

EP 0 001 821 B1

BUNDESDRUCKEREI BERLIN

Verfahren zur Racematspaltung von DL-α-Aminocarbonsäuren und dafür verwendete Salze

Die Erfindung betrifft ein Verfahren zur Herstellung einer optisch aktiven α-Aminocarbonsäure durch Racematspaltung einer DL-α-Aminocarbonsäure der Formel II

$$R-\underset{\underset{NH_2}{|}}{CH}-COOH \qquad (II)$$

in der R einen p-Hydroxyphenylrest, Phenylrest, Benzylrest oder einen iso-Propylrest bedeutet, durch Überführen in eine N-substituierte DL-α-Aminocarbonsäure und deren Salzbildung mit einer optisch aktiven Aminbase. Insbesondere betrifft die Erfindung ein Verfahren zur chemischen Spaltung racemischer Formen von α-Amino-4-hydroxy-phenyl-essigsäure.

α-Amino-4-hydroxyphenyl-essigsäure und deren Derivate sind wichtige Zwischenprodukte zur Herstellung von synthetischen Penicillinen und Cephalosporinen. Von den beiden möglichen Enantiomeren wird das D(−)-Isomere wegen seiner höheren pharmazeutischen Wirksamkeit in synthetischen Penicillin- und Cephalosporinverbindungen bevorzugt. Demzufolge hat die Herstellung von DL-α-Amino-4-hydroxy-phenyl-essigsäure und ihre Zerlegung in optische Antipoden sehr viel Interesse gefunden.

Verfahren zur Herstellung von DL-α-Amino-4-hydroxyphenyl-essigsäure und deren Derivaten sowie zur Spaltung derselben in die optischen Antipoden sind bereits bekannt.

So wird zum Beispiel in der britischen Patentschrift 1 240 687 die DL-Chloracetylamino-4-methoxyphenyl-essigsäure mit Schweinenierenacylase behandelt, wobei die L-Form selektiv deacetyliert wird. Die extraktiv abgetrennte D-Acylform wird in siedender Salzsäure hydrolysiert und mit Bromwasserstoffsäure demethyliert. In den deutschen Offenlegungsschriften 2 332 412 und 2 449 492 wird eine chemische Spaltung in die optischen Antipoden durch Salzbildung der N,O-Diacetyl- bzw. N-Benzoylaminosäure mittels (+)-α-Phenyläthylamin erreicht. Die kristallisierten Verbindungen werden nach Freisetzung der Aminbase in siedender Salzsäure verseift.

In der britischen Patentschrift 1 241 844 sowie in der deutschen Offenlegungsschrift 2 025 819 wird eine chemische Spaltung mit Hilfe von Chinintrihydrat beschrieben. Die Aminogruppe muß zuvor durch eine Carbobenzoxyfunktion geschützt werden. Die kristallisierte Fraktion kann nach der Freisetzung der optisch aktiven Base durch katalytische Hydrierung in die freie Aminosäure überführt werden.

In der deutschen Offenlegungsschrift 2 147 620 wird ein Racematspaltungsverfahren an dem N,O-Diacylderivat der DL-α-Amino-4-hydroxy-phenylessigsäure vorgeschlagen, das unter Verwendung von Dehydroabietylamin durchgeführt wird.

Ebenfalls an der N,O-Diacylverbindung wird in der deutschen Offenlegungsschrift 2 355 785 eine Racematspaltung mit Cinchonidin beschrieben. Die freie Aminosäure kann nach Behandlung mit siedender Salzsäure erhalten werden.

Im Biochem. J., 121, 425 (1971), schließlich wird eine Spaltung mit Bromcamphersulfonsäure beschrieben. Weiterhin ist aus der deutschen Offenlegungsschrift 2 309 180 bekannt, daß optisch aktive α-Aminosäureester durch Umsetzung eines Gemisches der enantiomeren Aminosäureester mit einer optisch aktiven Säure und einem Aldehyd, nämlich Benzaldehyd oder Anisaldehyd, hergestellt werden können, wobei ein Ester des gewünschten Enantiomeres in Salzform anfällt.

Alle bisher bekannten Methoden weisen jedoch eine Reihe von Nachteilen auf: Enzymatische Verfahren sind durch die schwierige Gewinnung und Reinigung der erforderlichen Enzyme sowie durch deren Instabilität sehr teuer und aufwendig. Chemische Racematspaltungsverfahren durch Bildung diastereomerer Salze aus N-substituierten Aminosäuren und optisch aktiven Aminbasen ergeben in den meisten Fällen nur niedrige Gesamtausbeuten an D-(−)-Aminosäuren. Die Komponenten sind häufig nur in großen Lösungsmittelmengen zu lösen, und die gebildeten diastereomeren Salze müssen oft durch Umkristallisationen aufgereinigt werden, um ausreichende optische Reinheit der resultierenden Aminosäure zu gewährleisten. Neben den hohen Preisen für die optisch aktiven Aminbasen muß als weiterer Nachteil die notwendige Einführung und Wiederabspaltung der N-Schutzgruppen angesehen werden, was Ausbeuteverluste zur Folge hat. Schließlich hat Bromcamphersulfonsäure, die ohne Deritvatisierung eingesetzt werden kann, den Nachteil, sehr teuer zu sein.

Aufgabe der Erfindung ist die Schaffung eines Verfahren zur chemischen Spaltung racemischer Formen von α-Amino-4-hydroxy-phenylessigsäure, das die obengenannten Nachteile nicht aufweist und dessen Prinzip sich verallgemeinert auf Racematspaltungen anderer α-Aminocarbonsäuren der Formel II anwenden läßt.

Die Erfindung betrifft ein Verfahren zur Herstellung einer optisch aktiven α-Aminocarbonsäure durch Racematspaltung einer DL-α-Aminocarbonsäure der Formel (II)

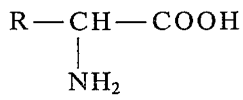

(II)

in der R einen p-Hydroxyphenylrest, Phenylrest, Benzylrést oder einen iso-Propylrest bedeutet, durch Überführen in eine N-substituierte DL-α-Aminocarbonsäure und deren Salzbildung mit einer optisch aktiven Aminbase und ist dadurch gekennzeichnet, daß

a) die DL-α-Aminocarbonsäure in einem für die Reaktionsteilnehmer inerten Lösungsmittel oder Lösungsmittelgemisch mit einem aromatischen Aldehyd der Formel (III)

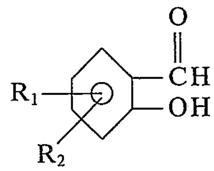

(III)

in der $R_1$ und $R_2$ gleich oder verschieden sind und jeweils ein Wasserstoffatom oder ein Halogenatom bedeuten, zu einem Azomethinderivat und dieses in der gleichen Reaktionsstufe mit einem optisch aktiven tertiären Amin mit einer Dissoziationskonstante von $10^{-9}$ bis $10^{-12}$ zu diastereomeren Salzen umgesetzt wird,

b) diese Salze dann durch fraktionierte Kristallisation getrennt werden und

c) aus den getrennten Salzen jeweils die optisch aktive α-Aminocarbonsäure mit einer starken Säure freigesetzt wird.

Azomethine aus aromatischen Aldehyden und Aminosäuren sind frühzeitig durch Arbeiten der Bergmann-Schule (M. Bergmann, H. Ensslin, L. Zervas, Chem. Ber., 58, 1034 [1925]) bekanntgeworden; sie wurden ausnahmslos in Form von Erdalkalimetall- oder Alkaloid-Salzen isoliert. Wieland et al. (T. Wieland, W. Schäfer, Ann. Chem., 576, 104 [1952]) gelang die Herstellung von N-Benzyliden-glycin-Kaliumsalz, das er über die gemischte Anhydridmethode weiter in den Thiophenylester umwandelte.

McIntire konnte erstmals zeigen, daß 5-Chlorsalicylaldehyd und 2-Hydroxynaphthaldehyd-(1) mit freien Aminosäuren stabile und gut kristallisierende Azomethine bilden (F. C. McIntire, J. Amer. Chem. Soc., 69, 1377 [1947]).

Die erfindungsgemäßen neuen Aminsalze unterscheiden sich von den in oben angegebenen Beispielen erwähnten Verbindungen dadurch, daß sie aus diastereomere Salze, die aus racemischen Gemischen von α-Aminosäuren erhalten werden, in ihre Stereoisomeren aufgetrennt werden können. Alkalimetallsalze, die aus racemischen Gemischen von α-Aminosäuren dargestellt werden, sind keine diastereomeren Salze und lassen sich nicht ohne chirales Hilfsmittel in ihre Stereoisomeren auftrennen. An den ebenfalls von Bergmann et al. (Chem. Ber., 58, 1034 [1925]) isolierten Alkaloidsalzen sind Auftrennungen in Diastereomere nicht beschrieben worden.

Das in der deutschen Offenlegungsschrift 2 400 489 dargestellte Verfahren zur Recamatspaltung über diastereomere Salze von optisch aktiven Aminbasen und Enaminderivaten von α-Aminocarbonsäuren hat gegenüber der vorliegenden Erfindung verschiedene Nachteile:

Die Ausbeuten im Salzbildungsschritt liegen bei 60–80% (bez. auf ein Enantiomeres der α-Aminosäure), im Einzelfall wird 83% nicht überschritten. Zur Erzielung günstiger Salzausbeuten sind zwecks Abscheidung des gebildeten Wassers erhöhte Temperaturen — meist Rückflußtemperatur des Lösungsmittels — erforderlich. Das nicht gewünschte Enantiomere der Aminosäure muß in einem zusätzlichen Arbeitsschritt nach üblichen Methoden — evtl. über die freie Aminosäure — racemisiert werden.

Das erfindungsgemäße Verfahren zeichnet sich dadurch aus, daß eine Derivatisierung der in seine optischen Antipoden zu spaltenden Aminosäure nicht erforderlich ist. Bei Zusammenbringen der Reaktanden Aminosäure, opt. akt. Amin und aromatischer Aldehyd in einem geeigneten Lösungsmittel wird bei guter Durchmischung bereits bei Raumtemperatur ohne Abtrennung des gebildeten Wassers die Entstehung einer Salzfällung beobachtet. Im Einzelfall kann die differentielle Löslichkeit der diastereomeren Salzpaare so groß sein, daß Ausbeuten über 95% (bez. auf ein Enantiomeres der α-Aminosäure) erzielt werden.

Die im erfindungsgemäßen Verfahren schon bei Raumtemperatur beobachtete Racemisierung am Chiralitätszentrum der Aminosäure des in Lösung befindlichen diastereomeren Salzes kann in unterschiedlicher Weise genutzt werden: Durch asymmetrische Transformation II. Art (E. E. Turner, Quart. Rev., 1947 [1], 299 ff.) lassen sich in diskontinuierlicher Arbeitsweise die Ausbeuten an gewünschtem diastereomeren Salz so weit steigern, daß praktisch 75% der eingesetzten racemischen

Aminosäure in die D-(−)-Form überführt werden können. Andererseits kann der in Lösung verbliebende Aminosäureanteil bei Temperaturen von etwa 20 bis max. 60°C ohne zusätzliche Operationen vollständig racemisiert und als DL-Verbindung zurückgewonnen werden. Sonst übliche Arbeitsgänge zur Racemisierung des erwünschten Enantiomeren entfallen.

Es ist überraschend, daß neben den Enaminsalzen auch mit den Azomethinsalzen der Aminosäuren — insbesondere am p-Hydroxy.phenylglycin — derart vollständig verlaufende Racematspaltungen unter Ausnutzung der differentiellen Löslichkeit diastereomerer Salzpaare möglich sind. Die ohne Abtrennung des Reaktionswassers schon bei Raumtemperatur beobachtete vollständige Umsetzung aller Komponenten bei gleichzeitiger Fällung eines diastereomeren Salzpaares war nicht zu erwarten. Besonders die leichte Racemesierbarkeit am Chiralitätszentrum des in Lösung befindlichen Azomethinderivates der Aminosäure überraschte, da bei vergleichbaren Racemisierungen an Aminosäurederivaten zusätzliche Aktivierung des Chiralitätszentrums durch Ester- oder Nitrilgruppen vorlag (Brit. Pat. 1 382 687 und DE-OS 2 309 180 über asymmetrische Transformationen an α-Aminonitrilen bzw. α-Aminosäureestern).

Die Herstellung der erfindungsgemäßen Salze wird gemäß dem folgenden Formelschema veranschaulicht:

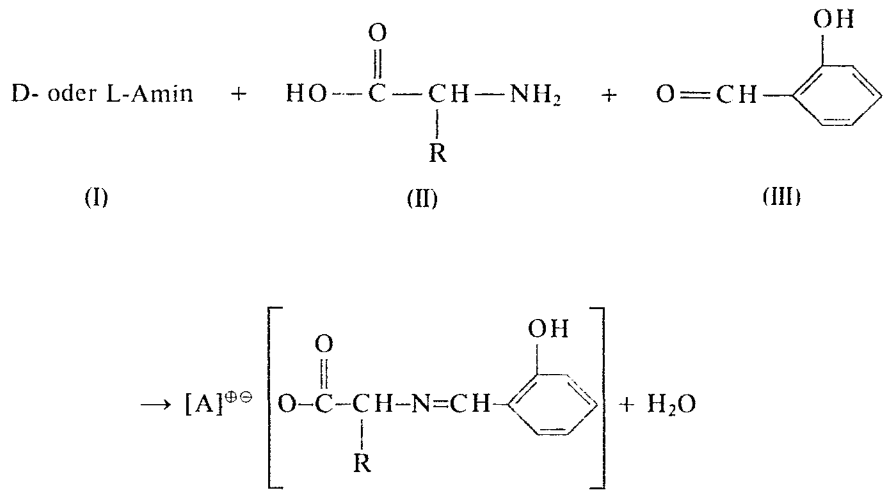


(I) (II) (III)

(IV)

worin A und R die in den Patentansprüchen formulierte Bedeutung besitzen.

Die Menge-Verhältnisse der Reaktionsteilnehmer (I), (II) und (III) sind stöchiometrisch, d. h., sie werden allgemein in äquimolaren Mengen eingesetzt. Vorzugsweise werden der verwendete aromatische Aldehyd (III) und die D- bzw. L-Aminbase (I) in einem 10−20%igen Überschuß über der Menge der α-Aminosäure (II) eingesetzt.

Die Reaktion läuft in einem breiten Temperaturbereich, d. h. von etwa 0°C bis zur Siedetemperatur des verwendeten Lösungsmittels zufriedenstellend ab. Vorzugsweise wird die Reaktion unter Rühren bei Raumtemperatur durchgeführt.

Das bei der Reaktion gebildete Wasser stört im Prinzip nicht, es kann jedoch unter Zuhilfenahme der üblichen Techniken aus dem Reaktionsgemisch entfernt werden.

Inerte Lösungsmittel, die im erfindungsgemäßen Verfahren angewandt werden, sind z. B. organische Lösungsmittel, welche nicht in die Reaktion eingreifen und in keiner Weise die gewünschte Richtung der Reaktion ändern.

Vorzugsweise eignen sich solche Lösungsmittel, welche die Auftrennung der diastereomeren Salze (IV) durch fraktionierte Kristallisation gestatten.

Beispiele für solche Lösungsmittel sind niedere Alkohole, Essigsäureäthylester, Acetonitril bzw. Dimethylformamid oder Gemische derselben. Äther und ähnliche unpolare Lösungsmittel sind als Verdünnungsmittel obengenannter Lösungsmittelsysteme geeignet.

Die Menge des jeweiligen Lösungsmittels kann experimentell leicht eingegrenzt werden. Die günstigsten Ausbeuten werden erhalten, wenn die eingesetzten Reaktionsteilnehmer nur langsam in Lösung gehen, während das Reaktionsprodukt bereits ausfällt.

Die Vervollständigung der vorstehend dargestellten Reaktion findet je nach Temperatur und Konzentration der Reaktanden allgemein in 1 Stunde bis 160 Stunden statt. Das Fortschreiten der Reaktion läßt sich leicht mit Hilfe konventioneller analytischer Methoden überwachen.

Nach Vervollständigung der Reaktion erhält man als Reaktionsprodukt ein Diastereomerengemisch von Salzen der Formel (IV).

Das Diastereomeren-Gemisch der Salze kann nach den üblichen Trennungsverfahren, bevorzugt jedoch durch fraktionierte Kristallisation, aufgetrennt werden. Wie dem Fachmann allgemein bekannt ist, ist eine Voraussage über die günstigsten Lösungsmittelsysteme, die eine Auftrennung durch fraktionierte Kristallisation gestatten, nicht mit Sicherheit möglich (E. L. Eliel, Stereochemie der Kohlenstoffverbindungen, Weinheim, 1966, S. 60 F.), und muß im Einzelfall durch experimentelle Daten in unterschiedlichen Lösungsmittelsystemen entschieden werden. Andere Techniken zur Trennung diastereomerer Gemische sind dem Fachmann bekannt (vgl. z. B. J. P. Greenstein, M. Winitz, »Chemistry of Amino Acids«, New York, 1961, Kap. 9).

Die als optisch aktive Aminbasen verwendeten Verbindungen sollten Dissoziationskonstanten im Bereich von etwa $10^{-9}$ bis $10^{-12}$ besitzen. Als besonders geeignet haben sich die D- bzw. L-Isomeren von tert. Aminen, wie N-Methylephedrin und 1-Phenyl-2-dimethyl-amino-propan-1,3-diol, erwiesen. Salzbildung kann jedoch auch mit einer Vielzahl anderer opt. aktiver, tertiärer Amine beobachtet werden. Einen Überblick über die am häufigsten verwendeten opt. aktiven Amine gibt S. H. Wilen in »Tables of Resolving Agents and Optical Resolution«, Notre Dame, 1972, Indiana.

Die zur Bildung der Azomethinderivate verwendeten aromatischen Aldehyde sollen zur Stabilisierung der Azomethinstruktur in ortho-Position mit einem Hydroxylrest substituiert sein (vgl. F. C. McIntire, J. Amer. Chem. Soc., 69, 1377 [1947]). Als besonders geeignet hat sich Salicylaldehyd erwiesen.

Wie aus den Beispielen ersichtlich ist, lassen sich nach dem erfindungsgemäßen Verfahren neben dem p-Hydroxyphenylglycin allgemein sowohl aliphatische als auch aromatische $\alpha$-Aminocarbonsäuren in die optischen Antipoden spalten.

Die nachfolgenden Beispiele dienen der Erläuterung der Erfindung, ohne diese einzuschränken:

### Beispiel 1

Herstellung eines Salzes aus l-N-Methylephedrin und N-Salicyliden-D-p-hydroxyphenylglycin in Methanol

Ein Gemisch aus 16,7 g (0,10 Mol) DL-p-Hydroxyphenylglycin, 20,6 g (0,115 Mol) l-N-Methylephedrin, 14,0 g (0,115 Mol) Salicylaldehyd und 120 ml Methanol wird bei 20–25°C etwa 64 h gerührt. Während des Rührvorganges tritt sofort intensive Gelbfärbung der Suspension ein. Nach etwa 1 h kann neben der Auflösung der festen Anteile die Kristallisation einer hellgelben Komponente beobachtet werden. Zur Vervollständigung der Fällung wird noch 1 h im Eisbad gerührt. Das Kristallisat wird abfiltriert und mit 30 ml Methanol-Äther-Gemisch (1 : 10) gewaschen. Nach dem Trocknen bei 50°C werden 21,5 g Salz von l-N-Methylephedrin und N-Salicyliden-D-p-hydroxyphenylglycin (95,4% der Theorie, bez. auf ein Enantiomeres) mit einer spez. Drehung $[\alpha]_D^{20} = +87,5°$ erhalten.

Für das noch nicht literaturbekannte Salz von l-N-Methylephedrin und N-Salicyliden-D-p-hydroxyphenylglycin wurden folgende Kennwerte ermittelt:

Fp.: 126–126,5°C (Methanol)
$[\alpha]_D^{20} = 91,6°$ (c=2, MeOH)

$^1$H-NMR (DMSO-$d_6$:
$\delta$ (TMS) 0,95 (d, 3; J=4,5 Hz); 2,64 (d, 6); 3,18 (m, 1); 5,08 (s, 1); 5,22 (d, 1; J=1,5 Hz); 6,7–7,5 (m, 13); 8,56 (d, 1); 8,0–9,5 (m, 4).

Elementaranalyse für $C_{26}H_{30}N_2O_5$:
ber.: C 69,3, H 6,7, N 6,2;
gef.: C 69,1, H 6,9, N 6,1.

In ähnlicher Weise wurde das folgende Beispiel ausgeführt, bei dem in einem Lösungsmittelgemisch von Methanol/Diäthyläther gearbeitet wurde. l-N-Methylephedrin und Salicylaldehyd wurden mit 15% Überschuß eingesetzt. Die Ausbeuteangabe bezieht sich auf ein Enantiomeres der Aminosäure. Die Fehlerbreite der spez. Drehungen ist mit etwa +0,5° anzusetzen.

### Beispiel 2

| | |
|---|---|
| Ansatzgröße: | 0,025 Mol |
| Rührzeit: | 24 Stunden |
| Lösungsmittel und Volumen: | 20 ml Methanol/20 ml Diäthyläther |
| Ausbeute: | 91,0% |
| $[\alpha]_D^{20}$ d. krist. Salzes: | +84,2° |

## Beispiel 3

### Herstellung eines Salzes aus l-N-Methylephedrin und N-Salicyliden-D-Valin in Essigsäureäthylester

Ein Gemisch aus 11,7 g (0,10 Mol) D,L-Valin, 20,6 g (0,115 Mol) l-N-Methylephedrin, 14,0 g (0,115 Mol) Salicylaldehyd und 180 ml Methanol wird bei Raumtemperatur etwa 1 h bis zur vollständigen Auflösung gerührt. Danach wird das Lösungsmittel unter vermindertem Druck entfernt und der ölige Rückstand mit 300 ml Essigsäureäthylester aufgenommen. Die klare Lösung wird mit einem Kristall des Salzes von l-N-Methylephedrin und N-Salicyliden-D-Valin geimpft, worauf sich nach kurzem Rühren bei Raumtemperatur ein gelber, kristalliner Niederschlag bildet. Das Kristallisat wird abfiltriert und mit wenig Essigsäureäthylester gewaschen. Nach dem Trocknen bei 50°C werden 5,65 g Salz von l-N-Methylephedrin und N-Salicyliden-D-Valin mit $[\alpha]_D^{20} = -49,2°$ erhalten.

Nach Einengen auf das halbe Volumen und erneutes Impfen der Lösung lassen sich nochmals 8,60 g Salz isolieren, die eine spez. Drehung $[\alpha]_D^{20}$ $-48,0°$ zeigen. Die Gesamtausbeute des Salzes von l-N-Methylephedrin und N-Salicyliden-D-Valin beträgt damit 71,2% der Theorie.

Fp.: 129–130° C (Essigsäureäthylester).

Elementaranalyse für $C_{23}H_{32}N_2O_4$:
ber.: C 69,0%, H 8,1%, N 7,0%;
gef.: C 69,0%, H 8,2%, N 7,0%.

## Beispiel 4

### Herstellung eines Salzes aus l-N-Methylephedrin und N-Salicyliden-D-phenylalanin in Essigsäureäthylester

Ein Gemisch aus 12,4 g (0,075 Mol) D,L-Phenylalanin, 15,5 g (0,086 Mol) l-N-Methylephedrin, 10,5 g (0,086 Mol) Salicylaldehyd und 135 ml Methanol wird bei Raumtemperatur etwa 2 h bis zur vollständigen Auflösung gerührt. Danach wird das Lösungsmittel unter vermindertem Druck entfernt und der ölige Rückstand mit 170 ml Essigsäureäthylester aufgenommen. Die klare Lösung wird mit einem Kristall des Salzes von 1-N-Methylephedrin und N-Salicyliden-D-phenylalanin geimpft, woraus sich nach kurzem Rühren bei Raumtemperatur ein gelber, kristalliner Niederschlag bildet. Das Kristallisat wird sofort abfiltriert und mit wenig Essigsäureäthylester gewaschen. Nach dem Trocknen bei 50° C werden 6,7 g (40% d. Theorie) Salz von l-N-Methylephedrin und N-Salicyliden-D-phenylalanin mit einer spez. Drehung von $[\alpha]_D^{20} = +107,4°$ erhalten.
Fp.: 119–120,5° C (Essigsäureäthylester).

Elementaranalyse für $C_{27}H_{32}N_2O_4$:
ber.: C 72,3%, H 7,2%, N 6,2%;
gef.: C 72,2%, H 7,2%, N 6,0%.

## Beispiel 5

### Herstellung eines Salzes gemäß Beispiel 1 mit anschließender asymmetrischer Transformation II. Art in Methanol

Zu 125 ml Methanol werden 16,7 g DL-Hydroxyphenylglycin (0,10 Mol), 20,6 g l-N-Methylephedrin (0,115 Mol) addiert und die Mischung anschließend 44 h bei 16–17° gerührt. Vor der Filtration des hellgelben Salzes wird noch etwa 1 h im Eisbad gerührt. Das abgetrennte Kristallisat wird mit 15 ml Äther/Methanol (10 : 1) gewaschen und bei 50° C im Trockenschrank getrocknet. Das blaßgelbe Salz zeigt eine spez. Drehung von $[\alpha]_D^{20} = +84,9$, die Ausbeute 20,2 g (89,7% d. Theorie, bez. auf ein Enantiomeres). Die Mutterlauge wird im Vakuum eingedampft und anschließend mit 40 ml Methanol aufgenommen. Man läßt 18 h bei Raumtemperatur stehen und kühlt dann für 6 h auf +6° C. Der ausgefallene Niederschlag wird wie oben abgetrennt, gewaschen und getrocknet. Das blaßgelbe Salz zeigt eine spez. Drehung von $[\alpha]_D^{20} = +86,7°$, die Ausbeute beträgt 3,0 g (13,3% der Theorie, bez. auf ein Enantiomeres). Die resultierende Mutterlauge wird wieder im Vakuum eingedampft und mit 20 ml Methanol aufgenommen. Man läßt 18 h bei Raumtemperatur stehen und kühlt dann für 4 h auf +6° C. Das ausgefallene blaßgelbe Salz wird wie oben behandelt, es zeigt eine spez. Drehung von $[\alpha]_D^{20}$ $+87,14°$, die Ausbeute beträgt 5,1 g (22,6% der Theorie, bez. auf ein Enantiomeres).

Abermals wird die Mutterlauge im Vakuum eingedampft und mit 10 ml Methanol aufgenommen. Nach 72 h bei Raumtemperatur wird 2 h auf +6° C gekühlt. Das ausgefallene Salz wird mit 40 ml Äther/Methanol (10 : 1) gewaschen und bei 50° C getrocknet. Die spez. Drehung beträgt $[\alpha]_D^{20} = +90,1°$,

die Ausbeute liegt bei 5,2 g (23,1% d. Theorie, bez. auf ein Enantiomeres). Gesamtausbeute 33,5 g (148,7% der Theorie, bez. auf ein Enantiomeres).

## Beispiel 6

### Herstellung eines Salzes aus d-threo-1-Phenyl-2-dimethyl-amino-propan-1,3-diol und N-Salicyliden-D-p-hydroxyphenylglycin in Methanol

Ein Gemisch aus 16,7 g (0,10 Mol) DL-p-Hydroxyphenylglycin, 22,4 g (0,115 Mol) d-threo-1-Phenyl-2-dimethylaminopropan-1,3-diol, 14,0 g (0,115 Mol) Salicylaldehyd und 80 ml Methanol wird bei Raumtemperatur unter Rühren durchmischt. Während des Rührvorganges tritt sofort intensive Gelbfärbung der Suspension ein, die nach etwa 1 h vollständig aufgelöst ist. Die klare Lösung wird mit einem Kristall des Salzes von d-Threo-1-Phenyl-2-dimethylaminopropan-1,3-diol und N-Salicyliden-D-p-Hydroxyphenyl-glycin geimpft und nach Zusatz von 80 ml Wasser etwa 17 h zur Vervollständigung der Fällung bei Raumtemperatur gerührt. Das hellgelbe Kristallisat wird abfiltriert, mit wenig Methanol-Äther-Gemisch (1 : 10) gewaschen und bei 50° C getrocknet. Die Ausbeute beträgt 21,5 g Salz von d-threo-1-Phenyl-2-dimethylamino-propan-1,3-diol und N-Salicyliden-D-p-Hydroxyphenylglycin (92,2% d. Theorie, bez. auf ein Enantiomeres) mit einer spez. Drehung von $[\alpha]_D^{20} = +115,9°$.

## Beispiel 7

### Spaltung des Salzes aus Beispiel 1 und Isolierung von D(−)-p-Hydroxyphenylglycin

Eine Lösung von 20,2 g (0,045 Mol) des Salzes von l-N-Methylephedrin und N-Salicyliden-D-p-hydroxyphenylglycin (Beispiel 1) in 100 ml Salzsäure (7,5%ig) werden zur Abtrennung des Salicylaldehyds dreimal mit je 50 ml Methylenchlorid extrahiert. Die verbleibende wäßrige Phase wird im Vakuum auf etwa 40 ml eingeengt und mit 20 ml Wasser verdünnt. Nach Erwärmen auf etwa 60−80° C wird mit etwa 16 ml Ammoniak (25%ig) auf pH 6,8 eingestellt und das ausgefallene p-Hydroxyphenylglycin abfiltriert. Man isoliert 5,39 g (71,9% d. Theorie) D(−)-p-Hydroxyphenylglycin mit einer spez. Drehung $[\alpha]_D^{20} = -154,6°$ (96,6% opt. Reinheit) (c=1; 6 n HCl). Die wäßrige Mutterlauge wird mit 85 ml Natronlauge (10%ig) auf pH 12 eingestellt. Der dabei entstehende, helle Niederschlag wird abgesaugt und mit wenig Ammoniakwasser (10%ig) gewaschen. Man isoliert 7,0 g (87,6% der Theorie) l-N-Methylephedrin mit praktisch unveränderter spez. Drehung.

Zur Nachfällung der Aminosäure wird das wäßrige Filtrat auf 50 ml eingeengt, mit 5 ml konz. Salzsäure auf pH 6,8 gestellt und über Nacht zur Kristallisation stehengelassen. Es werden nochmals 1,36 g D(−)-p-Hydroxyphenylglycin mit einer spez. Drehung $[\alpha]_D^{20} = -135,5°$ (84,7% opt. Reinheit) isoliert. Damit ergibt sich eine Gesamtausbeute von 90,0% Hydroxyphenylglycin mit einer opt. Reinheit von 94%.

## Beispiel 8

### Spaltung des Salzes aus Beispiel 3 und Isolierung von D(−)-Valin

Eine Lösung von 13,5 g (0,034 Mol) des Salzes von l-N-Methylephedrin und N-Salicyliden-D-Valin (Beispiel 3) in 40 ml Salzsäure (10%ig) werden zur Abtrennung des Salicylaldehyds dreimal mit je 25 ml Methylenchlorid extrahiert. Die verbleibende wäßrige Phase wird mit Natronlauge (35%ig) auf pH 12,5 gestellt und das dabei auskristallisierende Methylephedrin durch Filtration entfernt. Anschließend wird das ausgefallene Valin abfiltriert. Nach dem Trocknen werden 2,1 g (53% d. Theorie) D(−)-Valin in Form von schuppenförmigen, farblosen Kristallen mit einem Schmelzpunkt von 302° C, $[\alpha]_D^{20} = -26°$ (c=1; 6 n HCl) erhalten.

## Beispiel 9

### Spaltung des Salzes aus Beispiel 4 und Isolierung von D(+)-Phenylalanin

Eine Lösung von 5,85 g (0,013 Mol) des Salzes von l-N-Methylephedrin und N-Salicyliden-D-Phenylalanin (Beispiel 4) in 25 ml Salzsäure (10%ig) werden zur Abtrennung des Salicylaldehyds dreimal mit je 25 ml Methylenchlorid extrahiert. Die verbleibende wäßrige Phase wird mit Natronlauge (35%ig) auf pH 12,5 eingestellt und das dabei auskristallisierende N-Methylephedrin durch Filtration entfernt. Anschließend wird die Lösung mit konz. Salzsäure auf pH 6,8 gestellt und das ausfallende Phenylalanin abfiltriert. Nach dem Trocknen werden 1,3 g (60% der Theorie) D(+)-Phenylalanin als farblose Kristalle $[\alpha]_D^{20} = +16,7°$ (c=2, $H_2O$) erhalten.

Beispiel 10

Spaltung des Salzes aus Beispiel 6 und Isolierung von D(−)-p-Hydroxyphenylglycin

Eine Suspension von 22,5 g (0,0475 Mol) des Salzes von d-threo-1-Phenyl-2-dimethyl-amino-propan-1,3-diol und N-Salicyliden-D-p-Hydroxyphenylglycin (Beispiel 6) in 130 ml Wasser wird tropfenweise bis zur vollständigen Auflösung des Niederschlags bei pH 0,35 mit 10 ml Salzsäure (37%ig) versetzt. Der sich abscheidende Salicylaldehyd wird abgetrennt und die wäßrige Phase mit 75 ml (3×25 ml) Dichloräthan extrahiert. Die wäßrige Lösung wird am Rotationsverdampfer auf ein Volumen von 50 ml eingeengt und mit etwa 7 ml Ammoniak (25%ig) bei 40−60°C auf pH 6,8 eingestellt. Zur Vervollständigung der auftretenden Fällung wird etwa 1 h im Eisbad kaltgerührt. Das Kristallisat wird abgesaugt und bei 50°C getrocknet. Es werden 6,0 g (74,4% d. Theorie) D(−)-Hydroxyphenylglycin in Form von farblosen Kristallen mit einer spez. Drehung $[\alpha]_D^{20} = -150{,}3°$ (93,9% opt. Reinheit) (c=1; 6 n HCl) erhalten.
Fp. 227−229°C.

**Patentansprüche**

1. Verfahren zur Herstellung einer optisch aktiven α-Aminocarbonsäure durch Racematspaltung einer DL-α-Aminocarbonsäure der Formel (II)

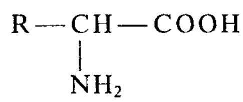

(II)

in der R einen p-Hydroxyphenylrest, Phenylrest, Benzylrest oder einen iso-Propylrest bedeutet, durch Überführen in eine N-substituierte DL-α-Aminocarbonsäure und deren Salzbildung mit einer optisch aktiven Aminbase, dadurch gekennzeichnet, daß

a) die DL-α-Aminocarbonsäure in einem für die Reaktionsteilnehmer inerten Lösungsmittel oder Lösungsmittelgemisch mit einem aromatischen Aldehyd der Formel (III)

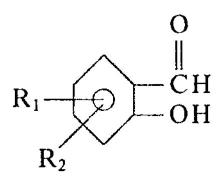

(III)

in der $R_1$ und $R_2$ gleich oder verschieden sind und jeweils ein Wasserstoffatom oder ein Halogenatom bedeuten, zu einem Azomethinderivat und dieses in der gleichen Reaktionsstufe mit einem optisch aktiven tertiären Amin mit einer Dissoziationskonstante von $10^{-9}$ bis $10^{-12}$ zu diastereomeren Salzen umgesetzt wird,

b) diese Salze dann durch fraktionierte Kristallisation getrennt werden und

c) aus den getrennten Salzen jeweils die optisch aktive α-Aminocarbonsäure mit einer starken Säure freigesetzt wird.

2. Verfahren nach Anspruch 1, dadurch gekennzeichnet, daß die Umsetzungen der Stufe a) bei einer Temperatur zwischen 0°C und der Siedetemperatur des Lösungsmittels durchgeführt werden.

3. Verfahren nach Anspruch 1, dadruch gekennzeichnet, daß die Umsetzungen der Stufe a) in einem Zeitraum von 1 bis 160 Stunden unter Rühren bei Raumtemperatur durchgeführt werden.

4. Verfahren nach Anspruch 1, dadurch gekennzeichnet, daß als inertes Lösungsmittel ein niederer aliphatischer Alkohol oder Essigsäureäthylester allein oder im Gemisch mit Wasser, Dimethylformamid oder Acetonitril eingesetzt wird.

5. Verfahren nach Anspruch 1, dadurch gekennzeichnet, daß als aromatischer Aldehyd o-Hydroxybenzaldehyd und als optisch aktives tertiäres Amin das D- oder L-Isomere von N-Methylephedrin oder threo-1-Phenyl-2-dimethylaminopropan-1,3-diol verwendet werden.

6. Verfahren nach Anspruch 1, dadurch gekennzeichnet, daß die Reaktionsteilnehmer der Stufe a) jeweils in äquimolaren Mengen eingesetzt werden.

7. Diastereomere Verbindung der Formel (IV)

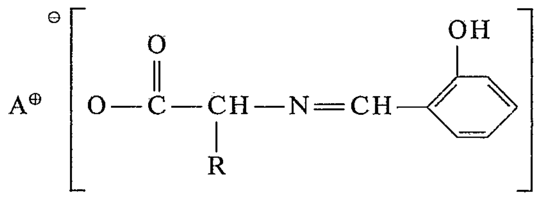

(IV)

worin A(+) ein durch Addition eines Protons an das Stickstoffatom eines optisch aktiven tertiären Amins mit einer Dissoziationskonstante von $10^{-9}$ bis $10^{-12}$ gebildetes Kation und R einen p-Hydroxyphenylrest, Benzylrest oder iso-Propylrest bedeuten.

8. Salz aus 1-N-Methylephedrin und N-Salicyliden-D-p-hydroxyphenylglycin.

9. Salz aus 1-N-Methylephedrin und N-Salicyliden-D-valin.

10. Salz aus 1-N-Methylephedrin und N-Salicyliden-D-phenylalanin.

11. Salz aus d-threo-1-Phenyl-2-dimethylamino-propan-1,3-diol und N-Salicyliden-D-p-hydroxyphenylglycin.

**Claims**

1. Process for the manufacture of an optically acitve $\alpha$-aminocarboxylic acid by splitting a racemic DL-$\alpha$-aminocarboxylic acid of the formula II

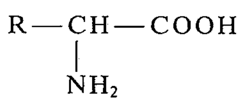

(II)

wherein R means a p-hydroxyphenyl groups, a phenyl group, a benzyl group or an isopropyl group, by conversion into a N-substituted DL-$\alpha$-aminocarboxylic acid and formation of a salt thereof with an optically acitve amine base, which comprises

a) reacting the DL-$\alpha$-aminocarboxylic acid, in a solvent or mixture of solvents being inert to the reaction participants, with an aromatic aldehyde of the formula III

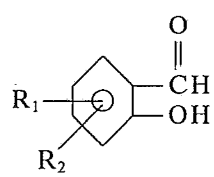

(III)

wherein $R_1$ and $R_2$ are identical or different and each mean a hydrogen atom or a halogen atom, to yield an azomethine derivative and reacting said azomethine derivative in the same reaction stage with an optical active tertiary amine having a dissociation constant of from $10^{-9}$ to $10^{-12}$ to yield diastereomeric salts,

b) separating said diastereomeric salts by fractional crystallization and

c) liberating the otically active $\alpha$-aminocarboxylic acid from the separated salts by means of a strong acid.

2. The process of claim 1, wherein the reactions of stage a) are carried out at a temperature between 0° C and the boiling temperature of the solvent.

3. The process of claim 1, wherein the reactions of stage a) are carried out at room temperature during a period of from 1 to 160 hours.

4. The process of claim 1, wherein a lower aliphatic alcohol or acetic acid ethyl ester either alone or in admixture with water, dimethyl formamide or acetonitrile is used as inert solvent.

5. The process of claim 1, wherein o-hydroxy benzaldehyde is used as aromatic aldehyde and the D-isomer or the L-isomer of N-methyl ephedringe or of threo-1-phenyl-2-dimethylamino-propane-1,3-diol is used as optically active tertiary amine.

6. The process of claim 1, wherein the reaction participants of stage a) are used each in equimolar amounts.

7. Diastereomeric compound of the formula IV

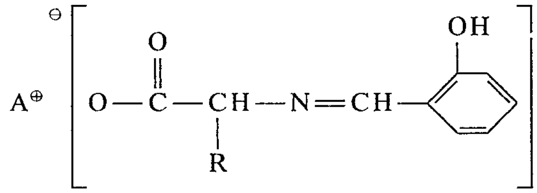

(IV)

wherein A(+) means a cation formed by addition of a proton to the nitrogon atom of an optically acitve tertiary amine having a dissociation constant of from $10^{-9}$ to $10^{-12}$ and R means a p-hydroxyphenyl group, a benzyl group or an isopropyl group.

8. Salt of 1-N-methyl ephedrine and N-salicyliden-D-p-hydroxyphenyl glycine.

9. Salt of 1-N-methyl ephedrine and N-salicyliden-D-valine.

10. Salt of 1-N-methyl ephedrine and N-salicyliden-D-phenyl alanine.

11. Salt of d-threo-1-phenyl-2-dimethylamino-propane-1,3-diol and N-salicyliden-D-p-hydroxyphenyl glycine.

**Revendications**

1. Procédé de préparation d'un acide α-amino-carboxylique optiquement actif par dédoublement du racémique d'un acide α-amino-carboxylique DL répondant à la formule II:

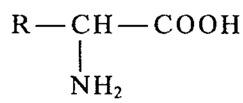

(II)

dans laquelle R représente un radical p-hydroxyphényle, phényle, benzyle ou isopropyle, par transformation en un acide α-amino-carboxylique DL substitué à l'azote et formation d'un sel de celui-ci avec une amine basique optiquement active, procédé caractérisé en ce que:

a) on fait réagir l'acide α-amino-carboxylique DL, dans un solvant ou mélange de solvants inertes à l'égard des corps participant à la réaction, avec un aldéhyde aromatique répondant à la formule III:

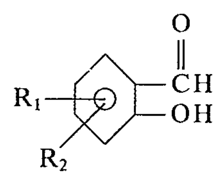

(III)

dans laquelle $R_1$ et $R_2$ représentent chacun, indépendamment l'un de l'autre, un atome d'hydrogène ou un atome d'halogène, et on fait réagir le composé azométhinique obtenu, dans la même étape réactionelle, avec une amine tertiaire optiquement active qui a une constante de dissociation comprise entre $10^{-9}$ et $10^{-12}$, réaction qui conduit à des sels diastéréo-isomères,

b) puis on sépare ces sels par cristallisation fractionnée et

c) de chacun des sels séparés on libère l'acide α-amino-carboxylique optiquement actif au moyen d'un acide fort.

2. Procédé selon la revendication 1, caractérisé en ce que les réactions des étapes a) sont effectuées à une température comprise entre 0° C et la température d'ébullition du solvant.

3. Procédé selon la revendication 1, caractérisé en ce que les réactions de l'étape a) sont effectuées dans un intervalle de temps de 1 à 160 heures, sous agitation, à la température ambiante.

4. Procédé selon la revendication 1, caractérisé en ce qu'on utilise, comme solvant inerte, un alcool aliphatique inférieur ou l'acétate d'éthyle, isolément ou en mélange avec l'eau, le diméthylformamide ou acétonitrile.

5. Procédé selon la revendication 1, caractérisé en ce qu'on utilise, comme aldéhyde aromatique, l'o-hydroxybenzaldéhyde et, comme amine tertiaire optiquement active, l'isomère D ou L de la N-méthyléphédrine ou du thréo-phényl-1 diméthylamino-2 propane-diol-1,3.

6. Procédé selon la revendication 1, caractérisé en ce que les partenaires réactionnels de l'étape a) sont utilisés en des quantités équimolaires.

7. Composé diastéréo-isomère répondant à la formule IV:

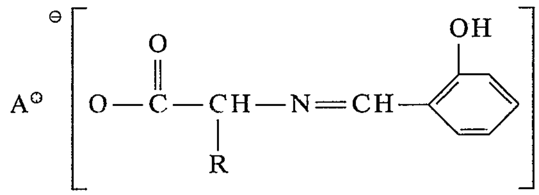

(IV)

dans laquelle $A^{(+)}$ représente un cation formé par addition d'un proton sur l'atome d'azote d'une amine tertiaire optiquement active ayant une constante de dissociation de $10^{-9}$ à $10^{-12}$ et R représente un radical p-hydroxyphényle, benzyle ou isopropyle.

8. Sel de N-méthyléphédrine 1 et de N-salicylidène-D-(p-hydroxyphényl)-glycine.

9. Sel de N-méthyléphédrine 1 et de N-salicylidène-D-valine.

10. Sel de N-méthyléphédrine 1 et de N-salicylidène-D-phénylalanine.

11. Sel de d-thréo-phényl-1 diméthylamino-2 propane-diol-1,3 et de N-salicylidène-D-(p-hydroxyphényl)-glycine.